# EUROPEAN PATENT APPLICATION

(11) **EP 4 767 952 A1**
(43) Date of publication of application: **01.07.2026**
(21) Application number: 24954601.1
(22) Date of filing: 30.09.2024
(51) Int. Cl.: A61B 17/22, A61B 18/12, A61M 25/00

(54) **SHOCKWAVE BALLOON DILATION CATHETER HAVING CORONA GENERATOR**

(30) Priority: 29.09.2024 CN 202411367635
(71) Applicant: Cyber-VP Medical Device (Shenzhen) Co., Ltd., Shenzhen, Guangdong 518122 (CN)
(72) Inventor: OUYANG, Junxiong, Guangdong 518122 (CN); LU, Lizhong, Guangdong 518122 (CN)
(74) Representative: Tahtadjiev, Konstantin
(86) International application number: PCT/CN2024/122603
(87) International publication number: WO 2026/065347

(57) **Abstract**

The present invention discloses a shockwave balloon dilation catheter with corona generator, including a dilation balloon, an outer tube, and an inner tube. The distal end of the inner tube extends beyond the distal end of the outer tube. The proximal end of the dilation balloon is sealed to the distal end of the outer tube, and the distal end of the dilation balloon is sealed to the distal end of the inner tube. A corona generator is disposed within the dilation balloon, including at least one set of electrodes each including at least one first electrode connected to a positive lead and at least one second electrode connected to a negative lead. The first and second electrodes are spaced apart. Discharge regions are provided on the first and second electrodes that contact the electrolyte liquid filling the inner cavity of the dilation balloon, thereby forming an electric field between the first and second electrodes. The first and second electrodes are fixed to the inner tube. Compared with conventional technologies, this invention improves the safety, ease of operation, reusability, and precise control of the balloon catheter.

## Description

### BACKGROUND OF THE PRESENT INVENTION

### FIELD OF INVENTION

The present invention relates to a medical device, and more particularly to a shockwave balloon dilation catheter with a corona generator based on liquid corona discharge.

### DESCRIPTION OF RELATED ARTS

In the field of medical devices, balloon catheters are mainly used to dilate narrowed blood vessels, and this treatment method has been proven to be sufficiently safe and effective. At present, balloon catheters are mainly based on the principle of arc discharge to inflate the balloon. Arc discharge is a discharge phenomenon in which a high-density plasma channel is formed through a dielectric under high voltage. Arc discharge occurs when, under high-voltage conditions, the dielectric between electrodes is completely broken down, forming a sustained high-temperature and high-density plasma channel. Arc discharge is a strong discharge phenomenon characterized by high temperature and high current density. Arc discharge is an overall effect in which the arc penetrates the dielectric between electrodes, forming a continuous high-temperature and high-current channel. Under a sufficiently high electric field, the liquid dielectric is completely broken down to form a high-density plasma channel, and this process is accompanied by intense energy release and the generation of high temperatures. The shock wave energy generated by arc discharge is enormous and difficult to control precisely, which may cause unpredictable damage to surrounding tissues. The shock wave effect of arc discharge is strong; although it can rapidly fragment calcified plaques, it imposes significant impact on surrounding vascular tissues, which may lead to vascular injury or other complications. The high-energy shock waves generated by arc discharge may result in high-risk complications such as vascular rupture and thrombosis. During the arc discharge process, the high temperature in the arc region may spread to surrounding tissues, causing unnecessary thermal damage. Moreover, the shock wave effect of arc discharge is typically a one-time strong effect, making it difficult to achieve multiple precise controls and cumulative therapeutic effects.

### SUMMARY OF THE PRESENT INVENTION

The present invention is to provide a shockwave balloon dilation catheter with a corona generator. The technical problem to be solved is to improve the safety, reusability and precise control of the balloon catheter.

To solve the above problems, the present invention adopts the following technical solution: A shockwave balloon dilation catheter with corona generator, comprising a dilation balloon, an outer tube, and an inner tube, the inner tube is disposed inside the outer tube, the inner tube and the outer tube are coaxial, a distal end of the inner tube is extended beyond a distal end of the outer tube, a proximal end of the dilation balloon is sealed to the distal end of the outer tube, and a distal end of the dilation balloon is sealed to the distal end of the inner tube; characterized in that: a corona generator is provided in the dilation balloon, and the corona generator comprises at least one set of electrodes each comprising at least one first electrode connected to a positive lead and at least one second electrode connected to a negative lead, wherein the first electrode and the second electrode in the set of electrodes are spaced apart, wherein the first electrode and the second electrode are respectively provided with discharge regions that contact the electrolyte liquid filled in an inner cavity of the dilation balloon so that an electric field is formed between the first electrode and the second electrode, wherein the first electrode and the second electrode are fixed on the inner tube.

Furthermore, the positive and negative leads are provided with exposed areas that are respectively connected to the first electrode and the second electrode, the exposed areas are respectively covered with metal electrode sleeves, and the metal electrode sleeves are respectively connected and fixed to the first electrode and the second electrode.

Furthermore, the first electrode and the second electrode are respectively ring-shaped, and grooves are respectively formed on edges of inner walls of the first electrode and the second electrode, and the metal electrode sleeves are respectively fixed on the grooves.

Furthermore, outer walls of the first electrode and the second electrode are respectively provided with a first insulating layer and a second insulating layer at rest areas except for the discharge regions, so as to expose the discharge regions.

Furthermore, the first electrode is provided with at least one first through hole, the second electrode is provided with at least one second through hole, the first through hole and the second through hole respectively constitute the discharge regions, the first insulating layer is completely covered on the rest area of the first electrode except for the first through hole, and the second insulating layer is completely covered on the rest area of the second electrode except for the second through hole, so as to form a directional electric field between the first electrode and the second electrode.

Furthermore, the discharge regions are respectively formed at annular edges of ends of the first electrode and the second electrode that are facing to each other, wherein the first insulating layer is completely covered on the first electrode except for the annular edge, and the second insulating layer is completely covered on the second electrode except for the annular edge, thereby forming a directional electric field between the first electrode and the second electrode.

Furthermore, the discharge regions are respectively formed at annular edges of ends of the first electrode and the second electrode that are facing away from each other, the first insulating layer is completely covered on the first electrode except for the annular edge, and the second insulating layer is completely covered on the second electrode except for the annular edge, thus forming a directional electric field between the first electrode and the second electrode.

Furthermore, the at least one set of electrodes comprises a single electrode set which comprises one first electrode and one second electrode.

Furthermore, the at least one set of electrodes comprises a single electrode set which comprises two first electrodes and one second electrode, wherein the second electrode is disposed between the two first electrodes.

Furthermore, the at least one set of electrodes comprises two electrode sets, wherein one electrode set comprises two first electrodes and one second electrode, and the other electrode set comprises one first electrode and one second electrode.

Compared with conventional technologies, the present invention, by incorporating the corona generator within the balloon, allows the corona generator to receive pulsed electrical signals from the power pulse generator. Under the influence of these pulsed signals, the electrolyte liquid undergoes a corona reaction, resulting in molecular ionization. The vapor bubbles generated by this ionization compress the electrolyte liquid, increasing the pressure within the balloon and causing it to expand radially along the inner tube. This improves the safety, ease of operation, reusability, and precise control of the balloon catheter.

### BRIEF DESCRIPTION OF THE DRAWINGS

FIG. 1 is a schematic view of the shockwave balloon dilation catheter of the present invention.
FIG. 2 is a cross-sectional view along the A-A direction in FIG. 1.
FIG. 3 is a cross-sectional view along the BB direction in FIG. 1.
FIG. 4 is a schematic view of the electrode assembly of the present invention.
FIG. 5 is another schematic view of the structure of the electrode assembly of the present invention.
FIG. 6 is a schematic view of the electric field of the first type of electrode assembly of the present invention.
FIG. 7 is a schematic view of the electric field of the second type of electrode assembly of the present invention.
FIG. 8 is a schematic view of the electric field of the third type of electrode assembly of the present invention.
FIG. 9 is a schematic view of the electric field of the fourth type of electrode assembly of the present invention.
FIG. 10 is a schematic view of the vapor bubbles generated by the electrode assembly of the present invention.
FIG. 11 is a graph showing the relationship between the area of the discharge region and the magnitude of the current in the present invention.
FIG. 12 is a graph showing the relationship between the electron path and the current magnitude in the present invention.
FIG. 13 is a schematic view of the structure of the present invention with two electrodes.
FIG. 14 is a circuit diagram of the present invention with two electrodes.
FIG. 15 is a schematic view of the structure of the present invention with three electrodes.
FIG. 16 is a circuit diagram of the present invention with three electrodes.
FIG. 17 is a schematic view of the structure of the two sets of electrodes of the present invention.
FIG. 18 is a circuit diagram of the two sets of electrodes of the present invention.

### DETAILED DESCRIPTION OF THE PREFERRED EMBODIMENT

The present invention will now be described in further detail with reference to the accompanying drawings and embodiments.

In the present invention, the distal end refers to the end furthest from the surgeon; the proximal end refers to the end closest to the surgeon.

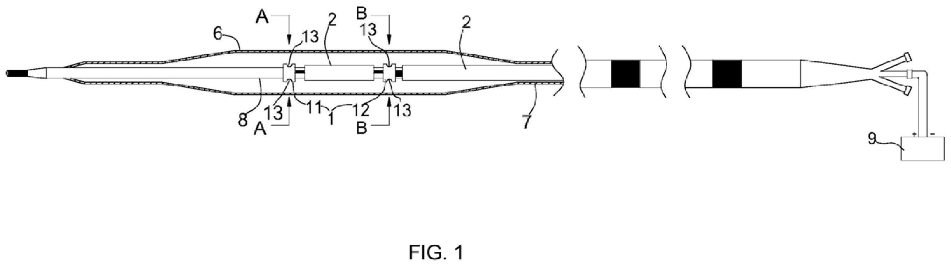

As shown in FIGS. 1 to 3, the present invention discloses a shockwave balloon dilation catheter with a corona generator, the shockwave balloon dilation catheter comprises a dilation balloon 6, an outer tube 7, and an inner tube 8. The inner tube 8 is disposed within the outer tube 7 and the two are coaxially arranged. The outer diameter of the inner tube 8 is smaller than the inner diameter of the outer tube 7, forming a fluid passage for electrolyte liquid. The distal end of the inner tube 8 extends beyond the distal end of the outer tube 7. The proximal end of the dilation balloon 6 is sealed to the distal end of the outer tube 7, and the distal end of the dilation balloon 6 is sealed to the distal end of the inner tube 7. The inner cavity of the dilation balloon 6 is communicated with the fluid passage. A corona generator is provided in the dilation balloon 6. The corona generator comprises at least one set of electrode group 1. Each set of electrode group 1 comprises at least one first electrode 11 connected to a positive lead and at least one second electrode 12 connected to a negative lead. The positive lead and the negative lead are electrically connected to the power pulse generator 9 via electrical connectors. The first electrode 11 and the second electrode 12 are provided with discharge regions 13 that are in contact with the electrolyte liquid filled in the inner cavity of the dilation balloon 6, so that an electric field is formed between the first electrode 11 and the second electrode 12. The corona generator receives the pulse electrical signal sent by the power pulse generator 9. Under the action of the pulse electrical signal, the electrolyte liquid undergoes a corona discharge through the discharge regions 13, and the electrolyte liquid produces molecular ionization. The vapor bubbles generated by molecular ionization are formed at the positions of the discharge regions 13, and squeeze the electrolyte liquid to increase the pressure inside the dilation balloon 6, causing the dilation balloon 6 to expand radially along the inner tube 8. The first electrode 11 and the second electrode 12 are fixed to the inner tube 8 by pasting or welding.

As shown in FIG. 1, a PET heat shrink tubing 2 can be fitted between the inner tube 8 and the lead wires to fix the lead wires to the inner tube 8. Alternatively, the inner tube 8 and the wire can be integrally formed by co-extrusion.

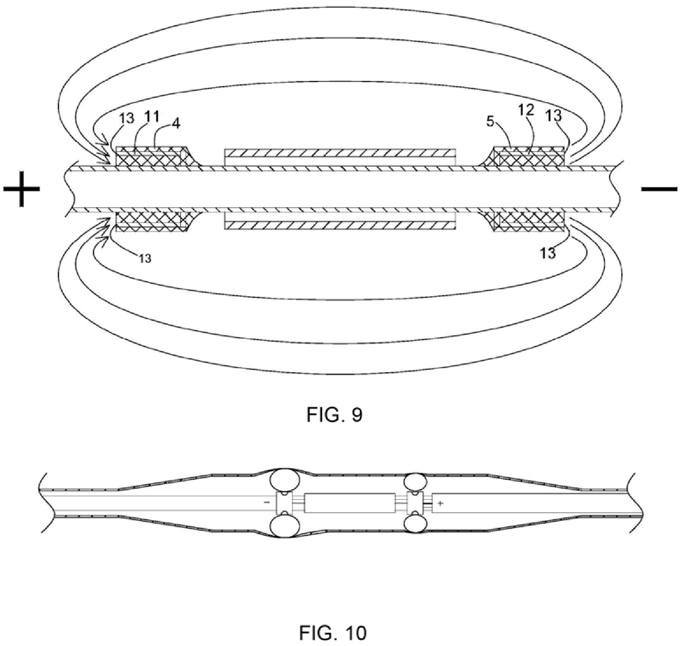

The corona generator is placed in the electrolyte liquid, the corona generator receives the pulse electrical signal sent by the power pulse generator 9, causing the electrolyte liquid to undergo a corona reaction, resulting in molecular ionization of the liquid, the plasma generated by molecular ionization vaporizes the surrounding liquid at high temperature, producing vapor bubbles. As the ionization reaction continues, the vapor bubble volume continuously expands and compresses the surrounding liquid environment. Due to the incompressible nature of the liquid, the internal pressure of the dilation balloon 6 is forced to increase instantaneously, causing the dilation balloon 6 to expand radially (as shown in FIG. 10), until the ionization reaction ends and the dilation balloon recovers, the time of the ionization reaction is determined by the pulse signal duration and pulse width parameters.

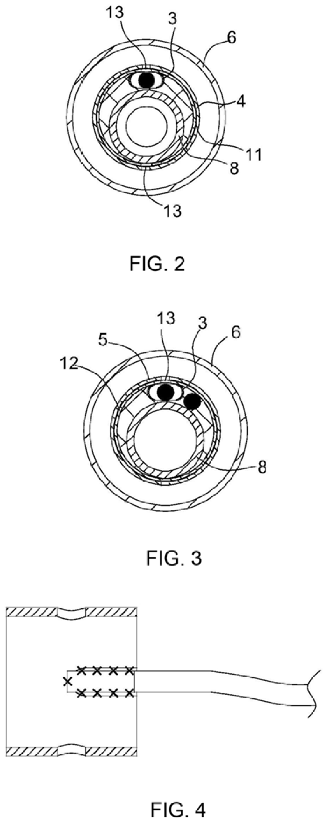

As shown in FIGS. 3 to 5, exposed areas are respectively provided on the positive and negative conductors and are respectively connected to the first electrode 11 and the second electrode 12. The rest areas of the positive and negative conductors are respectively covered with insulating layers. The positive and negative conductors can be covered with metal electrode sleeves 3 along the exposed areas of the inner tube. The metal electrode sleeves 3 are flat and are connected and fixed to the exposed areas by pasting or pressing, so that the metal electrode sleeves 3 are tightly connected to the exposed areas. The metal electrode sleeves 3 are respectively connected and fixed to the first electrode 11 and the second electrode 12 by welding.

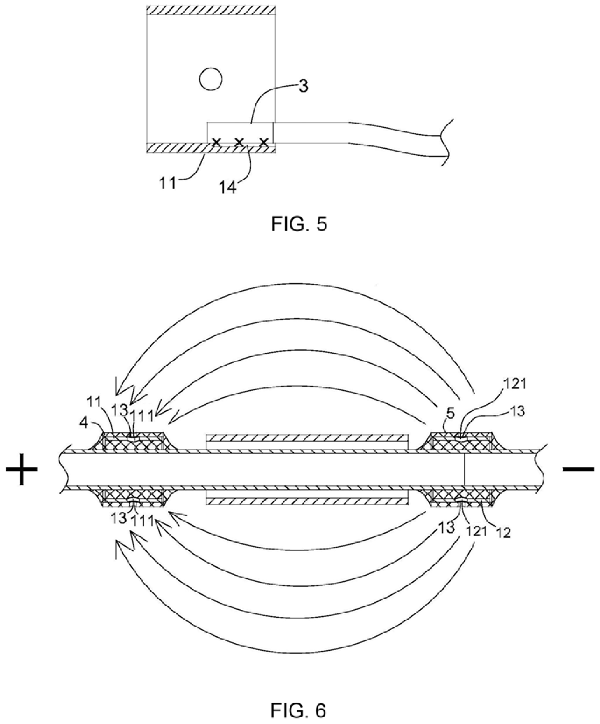

As shown in FIGS. 4 and 5, the first electrode 11 and the second electrode 12 in the present invention are ring shaped, specifically circular. FIGS. 4 and 5 illustrate the first electrode 11 as an example. Grooves 14 are respectively formed on the edges of the inner walls of the first electrode 11 and the second electrode 12. The metal electrode sleeve 3 is welded to the groove 14. Specifically, the overall size of the groove 14 can be smaller than the overall size of the metal electrode sleeve 3, so as to form a contact position on each of the first electrode 11 and the second electrode 12 that can be welded to the metal electrode sleeve 3, as shown in the " X " positions in FIGS. 4 and 5. The metal electrode sleeve 3 is connected to each of the first electrode 11 and the second electrode 12.

In the present invention, as shown in FIGS. 2 and 3, the outer walls of the first electrode 11 and the second electrode 12 are respectively provided with a first insulating layer 4 and a second insulating layer 5 at positions other than the discharge regions 13, so as to expose the discharge regions 13 and insulate the other positions, thereby receiving the pulse electrical signal sent by the power pulse generator 9 at the electrodes and forming a directional electric field between the electrodes.

As shown in FIG. 6, the first electrode assembly structure of the present invention has at least one first through hole 111 on the first electrode 11 and at least one second through hole 121 on the second electrode 12. The first through hole 111 and the second through hole 121 respectively constitute the discharge regions 13. The first insulating layer 4 is completely covered on the first electrode 11 except for the first through hole 111, and the second insulating layer 5 is completely covered on the second electrode 12 except for the second through hole 121. This allows the first electrode 11 and the second electrode 12 to receive pulse electrical signals sent by the power pulse generator 9 and form a directional electric field between the electrodes. Under the action of the pulse electrical signals, the electrolyte liquid undergoes a corona reaction. The potential distribution direction of this electrode assembly structure is relatively concentrated, which helps to reduce heat generation. The electron passage path is relatively concentrated, the resistance is relatively large, the current is reduced, and the safety is increased. It also facilitates steady-state symmetrical pressurization inside the balloon, making the entire balloon expand outward uniformly.

The first insulating layer 4 and the second insulating layer 5 can reduce the contact area between the first electrode 11 and the second electrode 12 and the electrolyte liquid, and release charge in the controlled discharge regions 13.

As a preferred embodiment, multiple first through holes 111 can be symmetrically arranged on the first electrode 11, and multiple second through holes 121 can be symmetrically arranged on the second electrode 12. Two first through holes 111 and two second through holes 121 are respectively arranged in a one-to-one correspondence. The metal electrode sleeve can be arranged at any position on the inner ring wall of each of the first electrode 11 and the second electrode 12.

In this embodiment, the interval between the first through hole 111 on the first electrode 11 and the second through hole 121 on the adjacent second electrode 12 is set to 4-8mm.

As can be seen from FIG. 6, the electric field formed in this embodiment forms two regions of electric field between the two through holes of the two electrodes, from the second through hole 121 of the second electrode 12 toward the first through hole 111 of the first electrode 11. Under the action of the pulsed electric signal, the electrolyte liquid undergoes a corona reaction.

When the first electrode group forms an electric field, the generated vapor bubbles are distributed at the positions of the first through hole 111 and the second through hole 121.

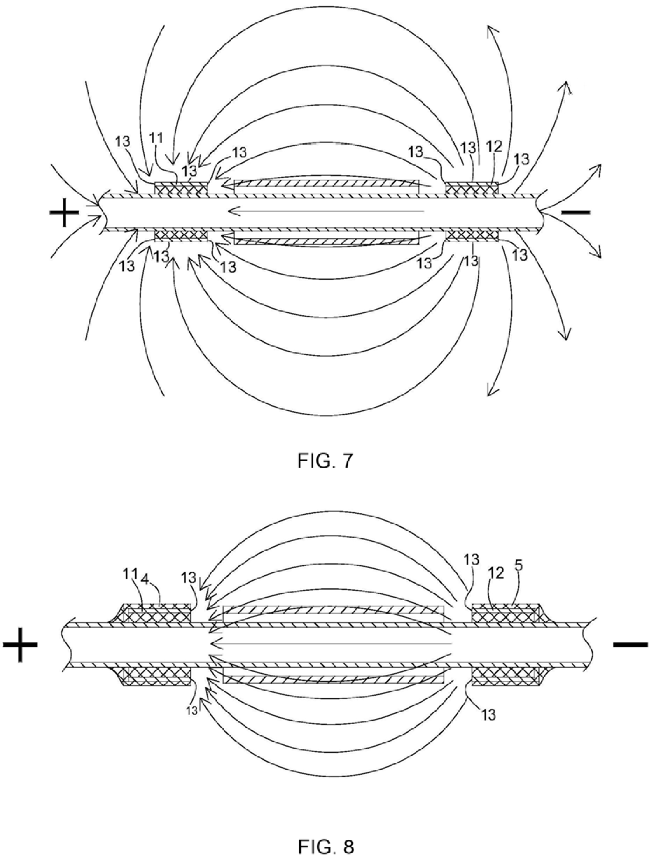

As a second electrode assembly structure of the present invention, as shown in FIG. 7, neither the first insulating layer 4 nor the second insulating layer 5 is wrapped around the first electrode 11 and the second electrode 12, so as to achieve an all-round electric field. FIG. 7 simulates the process of corona discharge from the second electrode 12 to the first electrode 11, and its electric field has divergence and a wider range.

When the second electrode group forms an electric field, the generated steam bubbles are uniformly distributed around the periphery of the entire first electrode 11 and the second electrode 12.

The second type of electrode structure can release charge outward over the largest area, forming a larger current and vapor bubbles. The charge distribution is more dispersed. Under the same excitation conditions, the pulse energy is larger, the bubble expansion volume is larger, the pressure inside the balloon is applied outward, and the outward expansion force is stronger. It is suitable for thicker, more stubborn calcifications.

As a third electrode assembly structure of the present invention, as shown in FIG. 8, the annular edges of the ends of the first electrode 11 and the second electrode 12 facing to each other form the discharge regions 13. The first insulating layer 4 is completely covered on the position of the first through hole 111 except for the annular edge, and the second insulating layer 5 is completely covered the position of the second electrode 12 except for the annular edge, so as to form an electric field between the facing ends of the first electrode 11 and the second electrode 12.

As shown in FIG. 8, since each discharge region 13 is annular, it will generate an electric field in the circumferential direction.

When the third type of electrode group forms an electric field, the generated steam bubbles will form around the facing annular edges of the two electrodes.

The third electrode group structure has the shortest potential direction path which enables vapor bubbles to be generated between the first electrode 11 and the second electrode 12. The vapor bubble is largest at the midpoint between the first electrode 11 and the second electrode 12. The generated force is conducted outward from between the first electrode 11 and the second electrode 12, and a large resultant force is generated in the middle of the first electrode 11 and the second electrode 12 to expand outward.

As a fourth electrode assembly structure of the present invention, as shown in FIG. 9, annular edges at ends of the first electrode 11 and the second electrode 12 facing away from each other form discharge regions 13. The first insulating layer 4 is completely covered on the position of the first through hole 111 except for the annular edge, and the second insulating layer 5 is completely covered on the position of the second electrode 12 except for the annular edge, so as to form an electric field between the sides of the first electrode 11 and the second electrode 12 facing away from each other.

As shown in FIG. 9, since the discharge regions 13 are respectively located far from the first electrode 11 and the second electrode 12, the directional electric field it forms is farther away than the directional electric fields of the first and third electrode group structures.

When the fourth electrode group forms an electric field, the generated vapor bubbles will form around the annular edges of the two electrodes on sides that are away from each other. The potential direction path of the fourth electrode group structure is the furthest, and the resulting force moves from the boundary of the expanding balloon towards the center. This causes the expanding balloon to preferentially expand outward from both the proximal and distal ends. This discharge method can reduce local concentration of the electric field, making the electric field distribution around the balloon more balanced, which is conducive to more stable energy transfer. At the same time, the preferential expansion of the balloon's sides can make the balloon expand more uniformly, reducing the phenomenon of uneven local expansion, and helping the balloon maintain a better shape and stability during expansion.

In the present invention, the electrode assembly is made of high-temperature resistant materials such as 304 stainless steel, 316 stainless steel, or tungsten-containing alloys. While ensuring excellent conductivity, the material can withstand the high-temperature plasma thermal erosion generated during corona discharge. The electrode assembly is spaced to prevent arcing between electrodes, which could lead to breakdown discharge. This space needs to be filled with electrolyte liquid to form a conductive path. Under certain parameters, a longer spacing results in a higher resistance in the conductive path and a smaller current.

FIG. 10 shows the bubbles generated by the corona discharge of the first electrode group structure. They are concentrated at the first through hole 111 of the first electrode 11 and the second through hole 121 of the second electrode 12. As the electrode energizing process continues, the vapor bubbles will continue to grow until they push the dilation balloon 6 to expand, thereby completing the treatment.

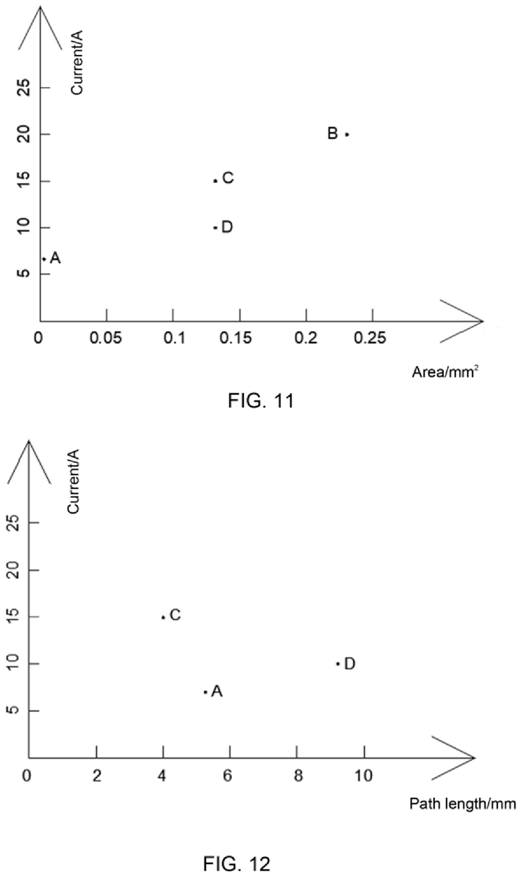

As shown in FIGS. 11 and 12, among the four electrode group structures mentioned above, the first is defined as A, the second as B, the third as C, and the fourth as D. It can be seen from the drawings that with the first electrode group structure, the discharge region is 0.002 mm² , the electron path length is 4.6 mm, and the current is 7 A. With the second electrode group structure, the total discharge region is 0.23 mm² , and the current is 20 A. With the third electrode group structure, the discharge region is 0.132 mm² , the electron path length is 4 mm, and the current is approximately 15 A. With the fourth electrode group structure, the discharge region is 0.132 mm² , the electron path length is 9.2 mm, and the current is 10 A.

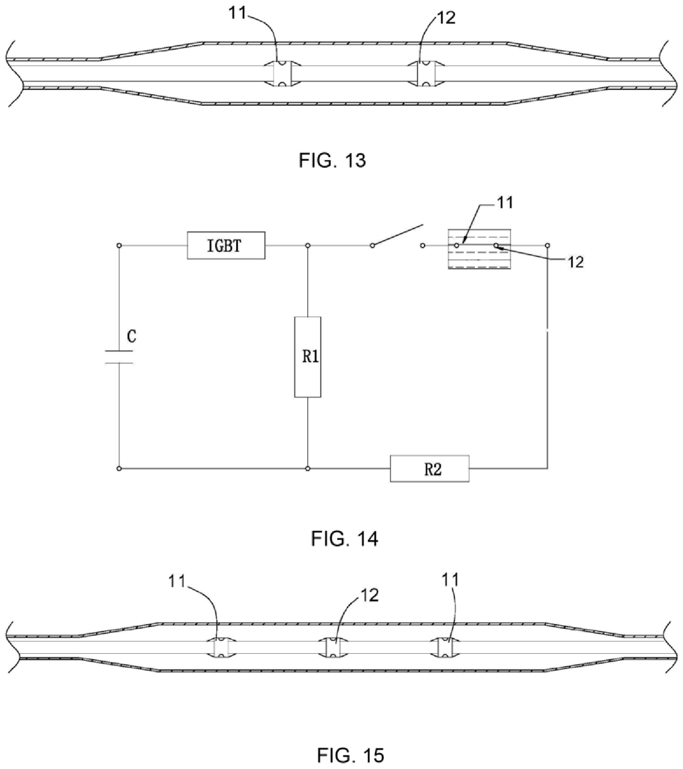

As shown in FIGS. 13 and 14, when the corona generator comprises one set of electrodes, the electrode set comprises one first electrode 11 and one second electrode 12. The first electrode 11 is connected to the positive terminal of the power pulse generator 9, and the second electrode 12 is connected to the negative terminal of the power pulse generator 9. The two electrodes receive the pulse electrical signal from the power pulse generator 9 and form an electric field between the discharge regions of the electrodes. Under the action of the pulse electrical signal, the electrolyte liquid undergoes a corona reaction.

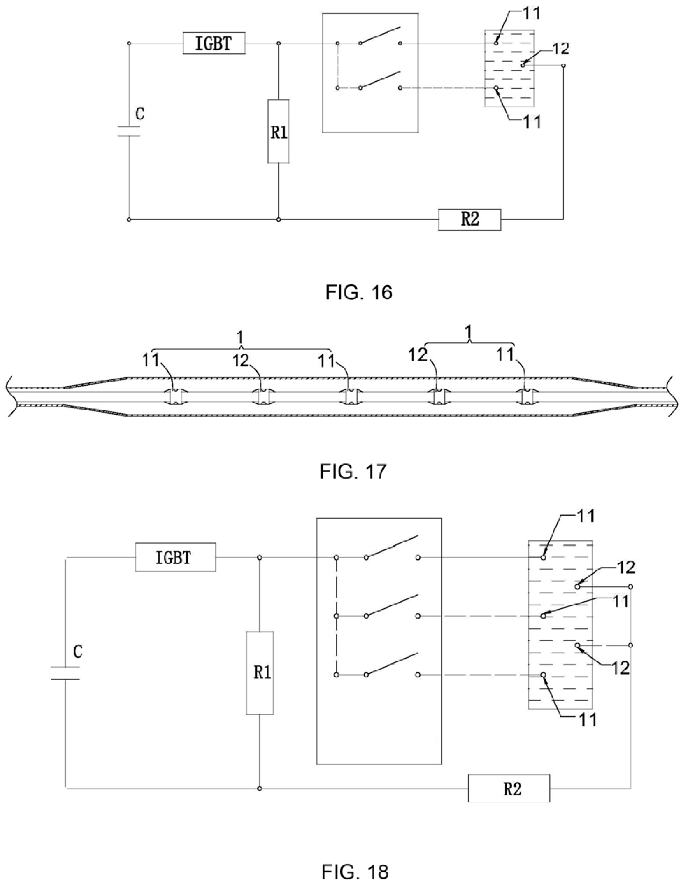

As shown in FIGS. 15 and 16, when the corona generator comprises one set of electrodes, the electrode set comprises two first electrodes 11 and one second electrode 12. The second electrode 12 is disposed between the two first electrodes 11 with equal spacing. The two first electrodes 11 are connected to the positive terminal of the power pulse generator 9, and the second electrode 11 is connected to the negative terminal of the power pulse generator 9. The two first electrodes 11 and the second electrode 12 receive the pulse electrical signal sent by the power pulse generator 9 and form an electric field between the discharge regions of the each two adjacent electrodes. Under the action of the pulse electrical signal, the electrolyte liquid undergoes a corona reaction.

As shown in FIGS. 17 and 18, when the corona generator comprises two sets of electrodes, one set of electrodes comprises two first electrodes 11 and one second electrode 12, and the other set of electrodes comprises one first electrode 11 and one second electrode 12. In the set of electrode groups 1 with three electrodes, the second electrode 12 is located between the two first electrodes 11, and the electrodes of the two sets of electrode groups are arranged at equal distances. The three first electrodes 11 are connected to the positive terminal of the power pulse generator 9, and the two second electrodes 12 are connected to the negative terminal of the power pulse generator 9. The two sets of electrode groups 1 receive the pulse electrical signals sent by the power pulse generator 4 and form electric fields between their respective electrodes, causing the electrolyte liquid to undergo a corona reaction under the action of the pulse electrical signals. Preferably, the first electrodes 11 and the second electrodes 12 in the two sets of electrode groups are arranged alternately.

As shown in FIGS. 14, 16, and 17, one end of the capacitor C in power pulse generator 9 is electrically connected to IGBT (Insulated Gate Bipolar Transistor). IGBT is divided into two paths: one path is electrically connected to one end of the first resistor R1, and the other path is electrically connected to the relay group K. The relay group K is electrically connected to the first electrode 11 which serves as the positive terminal. The other end of the first resistor R1 and the other end of the capacitor C are respectively electrically connected to one end of the second resistor R2. The other end of the second resistor R2 is electrically connected to the second electrode 12.

The capacitor C stores high-voltage charge. The first resistor R1 and the second resistor R2 are the working loads across the relay group K, protecting the circuit. When high-voltage charge needs to be released to the electrode, the circuit is switched on and off through the cooperation of IGBT (Insulated Gate Bipolar Transistor) and the relay group K. When one of the switches in relay group K is closed, the IGBT conducts, releasing the high-voltage charge stored in the capacitor C through the connecting circuit to form a high-voltage pulse. When multiple pairs of electrodes need to generate corona simultaneously or sequentially, multiple energy storage capacitors and multiple relays are used to manage the switching of the circuit.

The power pulse generator 9 can set the voltage value (1000-8000 V) and the pulse width value for releasing charge (1-200 us) in the circuit.

The present invention can be used to treat vascular stenosis and calcification. In use, under image guidance, a shockwave balloon dilation catheter (balloon dilation catheter) is introduced to the target site, i.e., the target vascular segment, through a guidewire. With the dilation balloon 6 not filled with electrolyte solution, the balloon dilation catheter can smoothly pass through the stenotic and calcified areas. Once the balloon dilation catheter reaches the target position, electrolyte fluid is filled into the dilation balloon 6 through the catheter system (catheter) to a preset pressure, causing it to adhere to the vessel wall and ensuring full contact between the dilation balloon 6 and the lesion area. A pulsed high voltage is applied, as shown in FIG. 10, causing a corona discharge from the corona generator. The corona discharge phenomenon causes the electrolyte fluid to vaporize, compressing the electrolyte fluid and increasing the pressure within the dilation balloon 6. The force continuously increases, thus transmitting pressure to the target site, breaking up calcified material and expanding the vascular lumen. Depending on the severity of the lesion and treatment needs, multiple corona discharges can be performed to ensure complete removal of the lesion. After each discharge, the dilation balloon 6 can be slightly drained, changing its diameter and adjusting the position of the balloon dilation catheter to cover the entire lesion area. After treatment, the internal fluid is drained, the dilation balloon 6 is retracted, and the balloon dilation catheter is withdrawn from the human body. Post-operative imaging examinations confirm vascular patency, demonstrating a good therapeutic effect. This approach effectively breaks up calcium in calcified lesions within blood vessels through controllable, instantaneous radial expansion of the balloon, preparing the vessel for subsequent treatment. Because arc discharge involves high temperatures in the arc area spreading to surrounding tissues, causing unnecessary thermal damage, this method utilizes corona discharge, significantly improving the effectiveness, safety, ease of operation, reusability, and precise control during interventional treatment of calcified blood vessels.

## Claims

1. A shockwave balloon dilation catheter with corona generator, comprising a dilation balloon (6), an outer tube (7), and an inner tube (8), the inner tube (8) is disposed inside the outer tube (7), the inner tube (8) and the outer tube (7) are coaxial, a distal end of the inner tube (8) is extended beyond a distal end of the outer tube (7), a proximal end of the dilation balloon (6) is sealed to the distal end of the outer tube (7), and a distal end of the dilation balloon (6) is sealed to the distal end of the inner tube (7); **characterized in that**: a corona generator is provided in the dilation balloon (6), and the corona generator comprises at least one set of electrodes (1) each comprising at least one first electrode (11) connected to a positive lead and at least one second electrode (12) connected to a negative lead, wherein the first electrode (11) and the second electrode (12) in the set of electrodes (1) are spaced apart, wherein the first electrode (11) and the second electrode (12) are respectively provided with discharge regions (13) that contact the electrolyte liquid filled in an inner cavity of the dilation balloon (6) so that an electric field is formed between the first electrode (11) and the second electrode (12), wherein the first electrode (11) and the second electrode (12) are fixed on the inner tube (8).

2. The shockwave balloon dilation catheter with corona generator according to claim 1, wherein the positive and negative leads are provided with exposed areas that are respectively connected to the first electrode (11) and the second electrode (12), the exposed areas are respectively covered with metal electrode sleeves (3), and the metal electrode sleeves (3) are respectively connected and fixed to the first electrode (11) and the second electrode (12).

3. The shockwave balloon dilation catheter with corona generator according to claim 2, wherein the first electrode (11) and the second electrode (12) are respectively ring-shaped, and grooves (14) are respectively formed on edges of inner walls of the first electrode (1) and the second electrode (12), and the metal electrode sleeves (3) are respectively fixed on the grooves (14).

4. The shockwave balloon dilation catheter with corona generator according to claim 3, wherein outer walls of the first electrode (11) and the second electrode (12) are respectively provided with a first insulating layer (4) and a second insulating layer (5) at rest areas except for the discharge regions (13), so as to to expose the discharge regions (13).

5. The shockwave balloon dilation catheter with corona generator according to claim 4, wherein the first electrode (11) is provided with at least one first through hole (111), the second electrode (12) is provided with at least one second through hole (121), the first through hole (111) and the second through hole (121) respectively constitute the discharge regions (13), the first insulating layer (4) is completely covered on the rest area of the first electrode (11) except for the first through hole (111), and the second insulating layer (5) is completely covered on the rest area of the second electrode (12) except for the second through hole (121), so as to form a directional electric field between the first electrode (11) and the second electrode (12).

6. The shockwave balloon dilation catheter with corona generator according to claim 4, wherein the discharge regions (13) are respectively formed at annular edges of ends of the first electrode (11) and the second electrode (12) that are facing to each other, wherein the first insulating layer (4) is completely covered on the first electrode (11) except for the annular edge, and the second insulating layer (5) is completely covered on the second electrode (12) except for the annular edge, thereby forming a directional electric field between the first electrode (11) and the second electrode (12).

7. The shockwave balloon dilation catheter with corona generator according to claim 4, wherein the discharge regions (13) are respectively formed at annular edges of ends of the first electrode (11) and the second electrode (12) that are facing away from each other, the first insulating layer (4) is completely covered on the first electrode (11) except for the annular edge, and the second insulating layer (5) is completely covered on the second electrode (12) except for the annular edge, thus forming a directional electric field between the first electrode (11) and the second electrode (12).

8. The shockwave balloon dilation catheter with corona generator according to one of claims 1-7, wherein the at least one set of electrodes comprises a single electrode set which comprises one first electrode (11) and one second electrode (12).

9. The shockwave balloon dilation catheter with corona generator according to one of claims 1-7, wherein the at least one set of electrodes comprises a single electrode set which comprises two first electrodes (11) and one second electrode (12), wherein the second electrode (12) is disposed between the two first electrodes (11).

10. The shockwave balloon dilation catheter with corona generator according to one of claims 1-7, wherein the at least one set of electrodes comprises two electrode sets, wherein one electrode set (1) comprises two first electrodes (11) and one second electrode (12), and the other electrode set (1) comprises one first electrode (11) and one second electrode (12).
